# EUROPÄISCHE PATENTSCHRIFT

(11) **EP 0 800 798 B1**
(45) Veröffentlichungstag und Bekanntmachung des Hinweises auf die Patenterteilung: **30.10.2002**
(21) Anmeldenummer: 97100735.6
(22) Anmeldetag: 17.01.1997
(51) Int. Cl.: A61C 5/06

(54) **Applikationsgerät für Dentalmasse**
Applicator device for dental material
Dispositif d'application pour substances dentaires

(30) Priorität: 10.04.1996 DE 29606540 U
(43) Veröffentlichungstag der Anmeldung: 15.10.1997
(73) Patentinhaber: Mühlbauer, Wolfgang Carl Friedrich, Dr., 22609 Hamburg (DE)
(72) Erfinder: Mühlbauer, Ernst, (DE)
(74) Vertreter: Glawe, Delfs, Moll & Partner

(56) Entgegenhaltungen:
- DE-A- 2 110 463
- DE-A- 3 109 333
- DE-C- 3 443 435
- US-A- 3 028 672
- US-A- 5 295 614

## Beschreibung

Die Erfindung bezieht sich auf ein Applikationsgerät für Dentalmasse, dessen langgestreckter Gerätekörper am vorderen Ende mit einer die Dentalmasse enthaltenden Spritze verbunden oder verbindbar ist und einen Stößel zum Vortreiben eines in der Spritze enthaltenen Kolbens aufweist, der mittels einer an dem hinteren Ende des Gerätekörpers herausragenden Drückerstange verschiebbar ist.

Bei bekannten Applikationstgeräten dieser Art (DE-C 25 28 116, DE-OS 21 10 465) sind Stößel und Drückerstange einstückig miteinander ausgeführt oder jedenfalls fest miteinander verbunden, so daß die Bewegung der Drückerstange sich unmittelbar auf den Stößel überträgt. Die das Geräte haltenden Finger bleiben in verhältnismäßig großer Entfernung vom behandelten Zahn und daher auch außerhalb des Mundes vom Patienten. Auch gibt die Art der Betätigung dem Zahnarzt ein sehr genaues Gefühl für die Dosierung der Dentalmasse. Schließlich sind viele Zahnärzte auch an diese Art der Betätigung gewöhnt, weil diese bekannten Geräte ihrer Einfachheit wegen während langer Zeit die einzigen waren, die für die Dosierung von Dentalmasse zur Verfügung standen. Sie haben jedoch den Nachteil, daß sie insbesondere bei zähfließenden Massen hohe Kräfte verlangen und aufgrund wechselnder Haftreibung keine ruckfreie Entleerung gestatten. Die Dosierung wird dadurch erschwert.

Es ist bekannt, derartige Geräte mit einem Hebelmechanismus zu versehen, der auf die aus dem Gerätekörper hinten herausragende Drückerstange einwirkt und wie eine Zange betätigt werden kann (US-A 4 198 756). Da diese Geräte in Fausthaltung betätigt werden, haben sie den Nachteil, daß ihre genaue Ausrichtung erschwert ist. Auch zeigen sie noch beträchtlichen Haftreibungs-Effekt.

Aus der US-5 295 614 ist für andere Anwendungsgebiete (z.B. den Wohnungsbau) ein Applikationsgerät für hochviskose Massen (z.B. Epoxidharze) bekannt, bei dem ein schwenkbarer Handhebel über eine Freilaufkupplung und ein Untersetzungsgetriebe auf eine Drückerstange einwirkt. Auch dieses Gerät wird in Fausthaltung betätigt. Zwar bewirkt die Kraftübersetzung des Getriebes eine Reduktion des Kraftaufwandes durch den Benutzer, wegen der Weguntersetzung ist zur Abgabe eines bestimmten Volumens aus der Spritze auch eine Mehrzahl von Arbeitsspielen des Schwenkhebels erforderlich. Bei Einhandbedienung wird die Ausrichtung eines solchen Geräts somit neben der bereits ungünstigen Fausthaltung noch durch die erforderliche Pumpbewegung der Hand verkompliziert. Da der Zahnarzt jedoch in der Regel nur eine Hand zur Bedienung frei hat, ist ein derartiges Applikationsgerät nicht für den Einsatz in der Zahnarztpraxis geeignet.

Bei einer anderen Gruppen von bekannten Geräten (USA 4 693 684, US-A 3 221 409, EP-B 237 182) ist am vorderen Ende des Geräts ein Hebel angeordnet, der mit dem Zeigefinger betätigt werden kann, wenn man das langgestreckte Gerät wie einen Bleistift hält, und der über eine Ratschenanordnung oder dergleichen auf den Stößel einwirkt. Abgesehen davon, daß diese Geräte kompliziert, vielteilig und entsprechend aufwendig sind, wird bei ihnen durch die Art der Betätigung eine Querkraft auf die Stößelstange ausgeübt, die deren Reibung im Gerätekörper und damit auch Haftreibungserscheinungen erhöht. Außerdem haben diese Geräte den Nachteil, daß die Betätigung nahe der Behandlungsstelle erfolgen muß und daher die Hand des Zahnarztes nicht immer außerhalb des Patientenmundes bleiben kann.

Zu der zuletzt erwähnten Gerätegattung gehört auch ein bekanntes Gerät (US-A 3 028 672), bei dem ein vorne am Gerätekörper angeordneter Betätigungsschieber mit einer Zahnstange verbunden ist, die über eine Serie von Zahnrädern mit dem gleichfalls eine Zahnstange tragenden Stößel verbunden ist. Der Sinn dieser Zahnradanordnung besteht darin, daß sie es ermöglicht, den vorderen, den Stößel enthaltenden Teil des Gerätekörpers schwenkbar gegenüber dem Griffteil des Gerätekörpers zu machen, wobei die Schwenkachse zwischen den beiden Teilen des Gerätekörpers zusammenfällt mit einer Zahnradachse. Da dieses im Bereich der Schwenkachse liegenden Zahnrad aus Platzgründen nicht unmittelbar mit den Zahnstangen des Schiebers und des Stößels zusammenwirken kann, sind noch vier weitere Übertragungszahnräder erforderlich. Eine Übersetzung ist nicht vorgesehen. Es liegt auf der Hand, daß diese Anordnung nicht nur kompliziert ist, sondern auch erhöhte Reibungsprobleme verursacht.

DE-A 2 110 463, das den Oberbegriff des Anspruchs bildet, offenbart eine Applikationsgerät für Dentalmasse, mit einer verschiebbaren Drückerstange und einem Stößel um die Dentalmasse zu enthalten und durch eine Spritze vorzutreiben.

Die Erfindung geht von der eingangs genannten Gattung von Applikationsgeräten aus und setzt sich zur Aufgabe, die Dosierung zu erleichtern. Die erfindungsgemäße Lösung besteht in den Merkmalen des Anspruchs 1.

Die Drückerstange wirkt nicht wie beim gattungsgleichen Stand der Technik unmittelbar auf den Stößel, sondern es ist ein Zahnradpaar zwischengeschaltet, das eine Untersetzung zwischen diesen beiden Teilen bewirkt, indem das größere Zahnrad mit der Zahnstange der Drückerstange und das kleinere Ritzel mit der Stößelzahnstange in Verbindung steht. Das Untersetzungsverhältnis wird durch das Durchmesserverhältnis von Zahnrad zu Ritzel bestimmt und liegt vorzugsweise zwischen 2:1 und 5:1, weiter vorzugsweise im Bereich von 3:1. Wenn eine stärkere Untersetzung verlangt wird, können auch mehrere Zahnradpaare vorgesehen sein.

Der Vorteil dieser Anordnung besteht darin, daß der Zahnarzt die ihm gewohnte Haltung und Bedienung des Geräts übernehmen kann und dennoch eine Übersetzung vorfindet, die den Kraftaufwand und die Reibungsprobleme herabsetzt. Es versteht sich, daß zu diesem Zweck das Ritzel und das Zahnrad koaxial drehfest miteinander verbunden und um eine feste Drehachse im Gerätekörper gelagert sind.

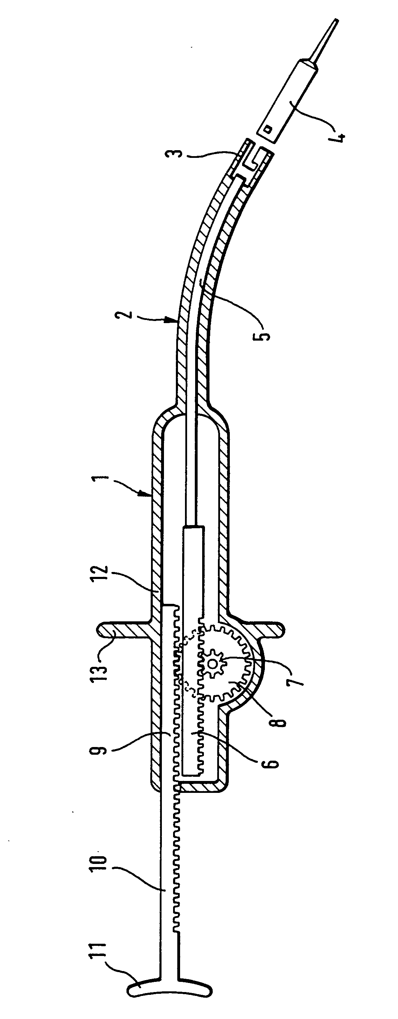

Die Erfindung wird im folgenden näher unter Bezugnahme auf die Zeichnung erläutert, die ein vorteilhaftes Ausführungsbeispiel veranschaulicht. Die einzige Figur zeigt einen Längsschnitt durch ein Applikationsgerät.

Der Gerätekörper besteht aus einem langgestreckten Hauptteil 1 und einem vorzugsweise ein wenig gebogenen vorderen, dünner werdenden Teil 2, der am Ende in einer Fassung 3 ausläuft, in der beispielsweise mittels einer bajonettartigen Befestigungsanordnung die Patrone 4 einer Spritze eingesetzt und gehalten werden kann, die die zu applizierende Masse und einen Kolben enthält. In dem vorderen Ende 2 des Gerätekörpers ist ein biegsamer Stößel 5 geführt, der in bekannter Weise mit dem Kolben in der Patrone 4 zusammenwirken kann, um diesen vorzutreiben. Er ist fest verbunden mit einer Stößelzahnstange 6, die in nicht dargestellter Weise im Hauptteil 1 dieses Gerätekörpers in ihrer Längsrichtung geführt ist und mit einem Ritzel 7 zusammenwirkt, dessen Welle im Gerätekörper in nicht dargestellter Weise gelagert ist. Das Ritzel 7 ist einstückig verbunden mit einem Zahnrad 8, das in eine Zahnstange 9 eingreift, die nach hinten zu einstückig in die Drückerstange 10 übergeht, die am Ende einen Betätigungsteller 11 trägt. Die Zahnstange 9 bzw. Drückerstange 10 ist ebenfalls in geeigneter Weise im Hauptteil 1 des Gerätekörpers geführt, beispielsweise einerseits durch dessen Wand 12 und andererseits durch den Umfang des Zahnrads 8 und die Öffnung, durch die Drückerstange 10 hinten aus dem Gerätekörper austritt. Die beiden Zahnstangen 6 und 9 verlaufen parallel zu einander in der Längsrichtung des Gerätekörpers.

Die Durchmesser des Zahnrads 8 und des Ritzels 7 verhalten sich zueinander etwa wie 3:1. Die Zahnstangen 6, 9 sind zur selben Seite hin am Zahnrad 8 bzw. Ritzel 7 angeordnet, so daß ihre Bewegung jeweils in derselben Richtung stattfindet, wobei die von der Drückerstange 10 vorgegebene Bewegung sich dem Stößel um das Untersetzungsverhältnis verringert mitteilt.

Wie ersichtlich, kann das Gerät ebenso gehalten und benutzt werden wie bekannte Geräte, bei denen die fest mit dem Stößel 5 verbundene Drückerstange 10 aus dem hinteren Ende des Gerätekörpers herausragt, indem es nämlich mit zwei Fingern vor dem Widerlager 13 und mit dem Daumen auf dem Teller 11 gefaßt wird. Der Unterschied besteht in der leichteren Dosierbarkeit und - infolge der Untersetzung - geringeren Empfindlichkeit gegenüber der Haftreibung des Stößels im Gerät und des Kolbens in der Patrone 4.

Die Erwähnung eines Zahnrad-Ritzelpaars im Anspruch mit unbestimmten Artikel soll nicht das Vorhandensein eines möglichen weiteren Zahnrad-Ritzelpaars für eine noch stärkere Untersetzung ausschließen. Die Drückzahnstange 9 wirkt dann mit dem Zahnrad des ersten Zahnrad-Ritzelpaars zusammen. Das Ritzel des ersten Zahnrad-Ritzelpaars treibt das Zahnrad des zweiten Zahnrad-Ritzelpaars an, dessen Ritzel auf die Stößelzahnstange 6 wirkt. Während in der Anordnung des Ausführungsbeispiels beide Zahnstangen auf derselben Seite des Zahnrad-Ritzelpaars angeordnet sind, müssen sie im Falle der Verwendung zweier Zahnrad-Ritzelpaare auf unterschiedlichen Seiten vorgesehen sein, um gleiche Bewegungsrichtung zu behalten.

## Patentansprüche

1. Applikationsgerät für Dentalmasse, dessen langgestreckter Gerätekörper (1,2) am vorderen Ende (3) mit einer die Dentalmasse enthaltenden Spritze (4) verbunden oder verbindbar ist und einen Stößel (5) zum Vortreiben eines in der Spritze (4) enthaltenen Kolbens aufweist, der mittels einer aus dem hinteren Ende des Gerätekörpers (1) herausragenden Drückerstange (10) verschiebbar ist, **dadurch gekennzeichnet, daß** der Stößel (5) und die Drückerstange (10) je mit einer Zahnstange (6,9) verbunden sind und zwischen den Zahnstangen (6,9) ein Zahnrad-Ritzelpaar (7,8) angeordnet ist, dessen Ritzel (7) mit der Stößelzahnstange (6) und dessen Zahnrad (8) mit der Betätigungszahnstange (9) zusammenwirkt.

## Claims

1. An applicator for a dental compound, whose elongate implement body (1, 2) is or can be connected at the front end (3) to a syringe (4) containing the dental compound and has a ram (5) for advancing a plunger contained in the syringe (4), which plunger can be displaced by means of a pusher rod (10) protruding out of the rear end of the implement body (1), wherein the ram (5) and the pusher rod (10) are each connected to a rack (6, 9), and a gearwheel/pinion pair (7, 8) is arranged between the racks (6, 9), the pinion (7) of which interacts with the ram rack (6), and the gearwheel (8) of which interacts with the actuating rack (9).

## Revendications

1. Dispositif d'application d'une masse dentaire dont le corps allongé (1, 2) est, ou peut être relié à son extrémité avant à une seringue (4) contenant la masse dentaire et qui présente un poussoir (5) pour actionner un piston contenu dans la seringue (4), qui peut être déplacé au moyen d'une tige de poussoir (10) dépassant de l'extrémité arrière du corps (1), **caractérisé par le fait que** le poussoir (5) et la tige de poussoir (10) sont chacun reliés à une crémaillère (6, 9) et qu'une paire de pignons dentés (7, 8) est disposée entre les crémaillères (6, 9) dont le pignon (7) coopère avec la crémaillère de poussoir (6) et le pignon (8) avec la crémaillère d'actionnement (9).
